(19) Europäisches Patentamt
European Patent Office
Office européen des brevets

(11) Publication number: **0 170 520 B1**

(12) **EUROPEAN PATENT SPECIFICATION**

(45) Date of publication of patent specification: **12.10.88**

(21) Application number: **85305420.3**

(22) Date of filing: **30.07.85**

# (54) Process for the production of cinnamic acid.

(30) Priority: **30.07.84 JP 161348/84**

(43) Date of publication of application: **05.02.86 Bulletin 86/06**

(45) Publication of the grant of the patent: **12.10.88 Bulletin 88/41**

(84) Designated Contracting States: **DE FR GB IT**

(56) References cited:
**GB-A- 782 430**

**CHEMICAL ABSTRACTS, vol. 81, no. 5, 5th August 1974, page 387, column 1, abstract no. 25099z, Columbus, Ohio, US; Y. WATANABE et al.: "Simultaneous production of acrylic acid and propylene oxide"; & JP - A - 73 42625 (SUMITOMO)**

**PATENT ABSTRACTS OF JAPAN, vol. 1, no. 13 (C-76)702r, 22th March 1977; & JP - A - 51 127 018 (ASAHI KASEI KOGYO) 11-05-1976**

(73) Proprietor: **SUMITOMO CHEMICAL COMPANY, LIMITED
15 Kitahama 5-chome Higashi-ku
Osaka-shi Osaka 541 (JP)**

(72) Inventor: **Harada, Haruhisa
7-23-5 Aobadai
Ichihara-shi Chiba-ken (JP)**

(74) Representative: **Geering, Keith Edwin et al
REDDIE & GROSE 16 Theobalds Road
London WC1X 8PL (GB)**

(56) References cited:
**PATENT ABSTRACTS OF JAPAN, vol. 6, no. 139 (C-116)1017r, 25th July 1982; & JP - A - 57 64640 (SANWA KAGAKU KOGYO) 19-04-1982**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 170 520 B1

Courier Press, Leamington Spa, England.

**Description**

The present invention relates to a process for producing cinnamic acid which is an industrially important compound, e.g. as a raw material for perfumes and L-phenylalanine.

Hitherto, cinnamic acid (abbreviated as CA hereinafter) has been produced by reacting benzaldehyde with acetic anhydride in the presence of potassium or sodium acetate, that is, by the Perkin process (Organic Reactions, Vol. 1, 217(1942)). This process is an established one but it has defects in that it employs a large amount of expensive acetic anhydride and potassium or sodium acetate, requires a precipitation step with an acid and a recrystallization step after the reaction, and produces a deal of waste water.

A process for the production of CA by the liquid-phase oxidation of cinnamic aldehyde (abbreviated as CAL hereinafter) prepared by the condensation of benzaldehyde with acetaldehyde in an aqueous caustic soda solution using molecular oxygen in the presence of silver oxide catalyst is a publicly-known process disclosed in US Patent No. 3162682 and in British Patent No. 782430. The latter process has the defects that it employs expensive silver oxide as the catalyst and requires a reaction temperature maintained at 30°C or lower (because a silver mirror reaction occurs, causing catalyst loss, if the reaction temperature exceeds 30°C). In addition, the said process requires a precipitation step with an acid and a recrystallization step and also produces a deal of waste water, as in the Perkin reaction.

We have found that, if CAL dissolved in an aromatic hydrocarbon is subjected to a liquid-phase oxidation reaction employing molecular oxygen in a reaction system containing said solution and coexisting water in the presence of a cobalt compound catalyst, CAL is converted into CA at a high yield, the reaction system is separated into an aqueous layer and an oil layer, and high-purity CA crystals are obtained readily by cooling the oil layer. Further, the aqueous layer preferably contains the catalyst dissolved in it and so can be recycled for re-use.

The invention is a process for the production of cinnamic acid by the oxidation of cinnamic aldehyde characterized in that cinnamic aldehyde dissolved in an aromatic hydrocarbon is oxidized in the liquid phase using molecular oxygen in the presence of cobalt catalyst and water.

As aromatic hydrocarbon for use in the method of the invention, those represented by the following general formula (I) are all usable but benzene, toluene, xylene, and polymethylbenzenes which resist an oxidizing atmosphere are particularly preferred.

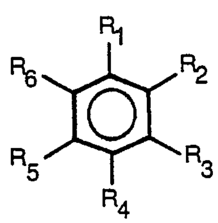

(I)

where $R_1$, $R_2$, $R_3$, $R_4$, $R_5$ and $R_6$ are each selected independently from hydrogen and alkyl groups of 1—4 carbon atoms.

The ratio of CAL to the said aromatic hydrocarbon varies with the solubility of the product CA in the said aromatic hydrocarbon but a concentration range of CAL of 10—50 wt% is preferred — because the oxidation reaction may be inefficient with a CAL concentration less than 10 wt% and the product CA may precipitate during the oxidation reaction with a CAL concentration exceeding 50 wt%. Characteristics of the method of the invention reside in the coexistence of water in the reaction system and in the use of cobalt compound as catalyst.

The coexistence of water results in improvement in selectivity for the product CA and permits easy separation of the catalyst when it is water soluble. A preferred range of the amount of coexisting water is 10—200 parts by weight based on 100 parts of aromatic hydrocarbon dissolving CAL, as separation of the catalyst may be difficult with an amount of coexisting water less than 10 parts by weight and an amount exceeding 200 parts by weight may be disadvantageous for product yield.

As the catalyst, all cobalt compounds are usable. Water-soluble cobalt compounds, however, are particularly preferable when separation of the catalyst is taken into account, and specific examples of water-soluble salts are cobalt acetate, cobalt benzoate, cobalt stearate, cobalt bromide, and the like. The amount of catalyst is preferably from 0.1—20% by weight as the concentration of an aqueous solution of the catalyst. When the said concentration is less than 0.1% by weight the catalytic effect may be low, and when the said concentration exceeds 20% by weight the conversion ratio of CAL and the selectivity for the product CA may be lowered. The aqueous phase containing the catalyst can be recycled for re-use.

The reaction temperature is preferably from 30—80°C; when the reaction temperature is lower than 30°C the reaction may be slow and product CA may be precipitated; when the reaction temperature exceeds 80°C, the selectivity for CA can be markedly lowered.

The reaction pressure is preferably from normal pressure to 20 kg/cm$^2$G.

By cooling the aromatic hydrocarbon solvent containing CA produced by the oxidation of CAL in

accordance with the method of the invention, and by further cooling the said aromatic hydrocarbon solvent after concentration as required, it has become possible to obtain high-purity CA crystals in one step without the necessity of performing precipitation with acid followed by recrystallization. Further, the oxidation reaction in the method of the invention can be performed either batchwise or continuously.

The invention is illustrated by the following Examples:

Example 1

15 g of CAL, 45 g of toluene, 60 g of water and 0.5 g of cobalt acetate were charged into a three-necked round-bottom flask having a capacity of 200 ml and the mixture was reacted at 40°C for 8 hours while blowing air into the reaction mixture at a rate of 100 cc/min. After completion of the reaction, the toluene phase was separated from the water phase without being cooled and then when the toluene phase was cooled, CA crystals precipitated. After being separated by filtration, the CA crystals were dried with air and the purity of CA crystals was determined by gas chromatography and by GPC★ measurement. The reaction results and the purity of CA are shown in Table-1. (★: Gel permutation chromatography)

Examples 2—5

Oxidation of CAL was carried out as in Example 1 except that instead of the cobalt acetate there was used cobalt benzoate (in Example 2), cobalt stearate (in Example 3), cobalt bromide (in Example 4) and cobalt (III) acetylacetonate (in Example 5). The results are shown in Table-1.

Comparative Example 1

Oxidation of CAL was carried out by the same method as in Example 1 except that no catalyst was used. The results are shown in Table-1.

The results of Table-1 indicate clearly that the method of the invention is superior to the oxidation reaction system employing no catalysts.

Table-1

| Example | Catalyst | CAL conv.(%) | CA select.(%) | CA crystal purity(%) |
|---|---|---|---|---|
| -1 | Co acetate | 53.6 | 80.1 | 99.8 |
| -2 | Co benzoate | 58.2 | 79.3 | 99.2 |
| -3 | Co stearate | 51.3 | 78.4 | 99.1 |
| -4 | Co bromide | 56.4 | 75.2 | 99.4 |
| -5 | Co acetyl-acetonate | 53.1 | 80.5 | 99.6 |
| Comparat. example-1 | No catalyst | 26.2 | 37.3 | 92.8 |

Examples 6—7

Oxidation of CAL was carried out in Examples 6 and 7 by the same method as in Example 1 except that, instead of toluene in Example 1, benzene was used (in Example 6) and p-xylene was used (in Example 7). The reaction results and the purity of the resulting CA crystals are shown in Table-2.

Comparative Example 2

15 g of CAL, 60 g of water, and 0.5 g of cobalt acetate were charged into a three-necked round flask and no aromatic hydrocarbon solvent was added to the reaction system. After that, oxidation of CAL was carried out by the same method as in Example 1. The results are shown in Table-2.

Comparative Example 3

15 g of CAL, 45 g of toluene, and 0.5 g of cobalt acetate were charged into a three-necked round flask and no water was added to the reaction system. After that, oxidation of CAL was carried out by the same method as in Example 1.

The results are shown in Table-2.

Table-2

| | Solvent | Presence of water | CAL conv.(%) | CA select.(%) | CA crystal purity(%) |
|---|---|---|---|---|---|
| Example-6 | Benzene | Present | 57.8 | 81.2 | 99.8 |
| Example-7 | p-xylene | Present | 56.2 | 80.5 | 99.1 |
| Comp.exa.-2 | No solvent | Present | 26.7 | 28.8 | Not precipitate |
| Comp.exa.-3 | Toluene | Not present | 52.8 | 32.5 | 80.5 |

The results of Table-2 indicate clearly the effect of coexistence of solvent and water.

Example 8

60 g of the separated water phase obtained in Example 1 was reused and the said separated water phase, 15 g of CAL, and 45 g of toluene were charged into a three-necked round flask. After that, oxidation of CAL was carried out by the same method as in Example 1. The reaction results and the purity of CA crystals obtained are shown in Table-3 and compared with results of Example 1.

Table-3

| | CAL conversion(%) | CA selectivity(%) | CA crystal purity(%) |
|---|---|---|---|
| Example-1 | 53.6 | 80.1 | 99.8 |
| Example-8 | 52.8 | 80.0 | 99.3 |

While the invention has been described in detail and with reference to specific embodiments thereof, it will be apparent to one skilled in the art that various changes and modifications can be made therein without departing from the spirit and scope thereof.

**Claims**

1. A process for the production of cinnamic acid by the catalytic liquid-phase oxidation of cinnamic aldehyde with molecular oxygen, characterised in that the oxidation is conducted in the presence of cobalt compound catalyst and water with the cinnamic aldehyde dissolved in aromatic hydrocarbon solvent selected from those of the formula

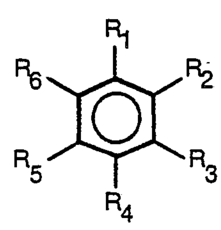

(I)

wherein each of $R_1$, $R_2$, $R_3$, $R_4$, $R_5$ and $R_6$ is selected independently from a hydrogen atom and alkyl groups of 1 to 4 carbon atoms.

2. A process according to claim 1 characterised in that the aromatic hydrocarbon solvent is selected from benzene, toluene, and xylene.

3. A process according to claim 1 or 2 characterised in that the cobalt compound catalyst is water soluble.

4. A process according to claim 3 characterised in that the cobalt compound catalyst is selected from cobalt acetate, cobalt benzoate, cobalt stearate and cobalt bromide.

5. A process according to any of claims 1 to 4 characterised in that the reaction temperature is from 30 to 80°C.

6. A process according to any of claims 1 to 5 characterised in that the reaction mixture contains from 10 to 200 parts by wt. of water per 100 parts by wt. of aromatic hydrocarbon solvent.

7. A process according to any of claims 1 to 6 characterised in that the initial concentration of cinnamic aldehyde in the aromatic hydrocarbon solvent is from 10 to 50 wt.%.

8. A process according to any of claims 1 to 7 characterised in that the reaction pressure is up to 20 kg/cm$^2$K.

9. A process according to any of claims 1 to 8 characterised in that the organic phase containing product cinnamic acid is separated from the aqueous phase.

**Patentansprüche**

1. Verfahren zur Herstellung von Zimtsäure durch katalytische Flüssigphasenoxidation von Zimtaldehyd mit molekularem Sauerstoff, dadurch gekennzeichnet, daß die Oxidation in Gegenwart einer Kobaltverbindung als Katalysator und Wasser durchgeführt wird, wobei der Zimtaldehyd in einem aromatischen Kohlenwasserstofflösungsmittel aufgelöst ist, ausgewählt aus einem solchen der Formel

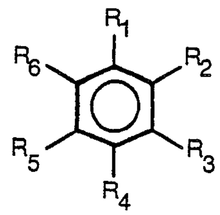

(I)

worin jeweils $R_1$, $R_2$, $R_3$, $R_4$, $R_5$ und $R_6$ unabhängig voneinander ausgewählt sind aus einem Wasserstoffatom und Alkylgruppen mit 1 bis 4 Kohlenstoffatomen.

2. Verfahren nach Anspruch 1, dadurch gekennzeichnet, daß das aromatische Kohlenwasserstofflösungsmittel ausgewählt ist aus Benzol, Toluol und Xylol.

3. Verfahren nach Anspruch 1 oder 2, dadurch gekennzeichnet, daß der aus einer Kobaltverbindung bestehende Katalysator wasserlöslich ist.

4. Verfahren nach Anspruch 3, dadurch gekennzeichnet, daß der aus einer Kobaltverbindung bestehende Katalysator ausgewählt wird aus Kobaltacetat, Kobaltbenzoat, Kobaltstearat und Kobaltbromid.

5. Verfahren nach einem der Ansprüche 1 bis 4, dadurch gekennzeichnet, daß die Reaktionstemperatur 30 bis 80°C beträgt.

6. Verfahren nach einem der Ansprüche 1 bis 5, dadurch gekennzeichnet, daß die Reaktionsmischung 10 bis 200 Gew.-Teile Wasser pro 100 Gew.-Teile aromatisches Kohlenwasserstofflösungsmittel enthält.

7. Verfahren nach einem der Ansprüche 1 bis 6, dadurch gekennzeichnet, daß die Anfangskonzentration des Zimtaldehyds in dem aromatischen Kohlenwasserstofflösungsmittel 10 bis 50 Gew.-% beträgt.

8. Verfahren nach einem der Ansprüche 1 bis 7, dadurch gekennzeichnet, daß der Reaktionsdruck bis zu 20 kg/cm$^2$K beträgt.

9. Verfahren nach einem der Ansprüche 1 bis 8, dadurch gekennzeichnet, daß die organische Phase, welche das Produkt Zimtsäure enthält, von der wäßrigen Phase abgetrennt wird.

**Revendications**

1. Procédé de production d'acide cinnamique par oxydation catalytique en phase liquide d'aldéhyde cinnamique avec de l'oxygène moléculaire, caractérisé en ce que l'oxydation est conduite en présence d'un composé de cobalt servant de catalyseur et d'eau, l'aldéhyde cinnamique étant dissous dans un solvant hydrocarboné aromatique choisi entre ceux de formule

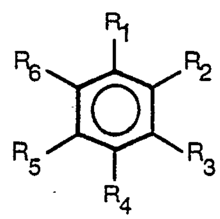

(I)

dans laquelle chacun des substituants $R_1$, $R_2$, $R_3$, $R_4$, $R_5$ et $R_6$ est choisi indépendamment entre un atome d'hydrogène et des groupes alkyle de 1 à 4 atomes de carbone.

2. Procédé suivant la revendication 1, caractérisé en ce que le solvant hydrocarboné aromatique est choisi entre le benzène, le toluène et le xylène.

3. Procédé suivant la revendication 1 ou 2, caractérisé en ce que le composé de cobalt servant de catalyseur est hydrosoluble.

4. Procédé suivant la revendication 3, caractérisé en ce que le composé de cobalt servant de catalyseur est choisi entre l'acétate de cobalt, le benzoate de cobalt, le stéarate de cobalt et le bromure de cobalt.

5. Procédé suivant l'une quelconque des revendications 1 à 4, caractérisé en ce que la température de réaction est comprise dans l'intervalle de 30 à 80°C.

6. Procédé suivant l'une quelconque des revendications 1 à 5, caractérisé en ce que le mélange réactionnel contient 10 à 200 parties en poids d'eau pour 100 parties en poids de solvant hydrocarboné aromatique.

7. Procédé suivant l'une quelconque des revendications 1 à 6, caractérisé en ce que la concentration initiale d'aldéhyde cinnamique dans le solvant hydrocarboné aromatique est comprise dans l'intervalle de 10 à 50% en poids.

8. Procédé suivant l'une quelconque des revendications 1 à 7, caractérisé en ce que la pression réactionnelle atteint 20 kg/cm$^2$ K.

9. Procédé suivant l'une quelconque des revendications 1 à 8, caractérisé en ce que la phase organique contenant l'acide cinnamique obtenu comme produit est séparée de la phase aqueuse.